# EUROPEAN PATENT APPLICATION

(11) **EP 2 439 274 A2**
(43) Date of publication of application: **11.04.2012**
(21) Application number: 10783611.6
(22) Date of filing: 04.06.2010
(51) Int. Cl.: C12N 15/11, C12N 15/63

(54) **MULTI-CISTRONIC shRNA EXPRESSION CASSETTE FOR SUPPRESSING SINGLE OR MULTIPLE TARGET GENES**

(30) Priority: 05.06.2009 KR 20090049920
(71) Applicant: Seol, Dai-wu, Gyeonggi-do 461-832 (KR)
(72) Inventor: Seol, Dai-wu, Gyeonggi-do 461-832 (KR)
(74) Representative: Graf von Stosch, Andreas
(86) International application number: PCT/KR2010/003599
(87) International publication number: WO 2010/140862

(57) **Abstract**

The present invention relates to one multi-cistronic shRNA for suppressing single or multiple target genes, and more particularly, to a multi-cistronic shRNA expression cassette, an expression vector comprising the multi-cistronic shRNA expression cassette, cells transduced with the multi-cistronic shRNA expression vector, a composite of the expression vector and a carrier, a method for suppressing various target genes, and a composition for suppressing target genes comprising the expression vector.

## Description

### BACKGROUND OF THE INVENTION

### 1. Field of the Invention

The present invention relates to a multi-cistronic shRNA for suppressing single or multiple target genes, and more particularly, to a multi-cistronic shRNA expression cassette, an expression vector comprising the multi-cistronic shRNA expression cassette, a cell transduced with the multi-cistronic shRNA expression vector, a composite of the expression vector and a carrier, a method for suppressing various target genes, and a composition for suppressing target genes comprising the expression vector.

### 2. Description of the Related Art

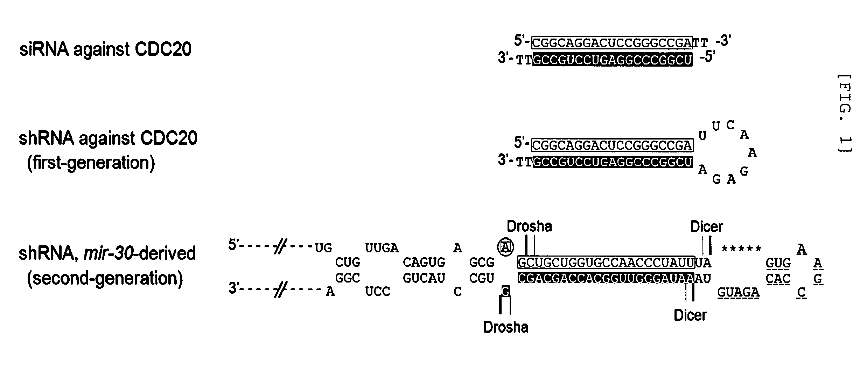

RNA interference (hereinafter, referred to as RNAi) refers to a phenomenon of selectively inducing degradation of target mRNAs and target gene silencing by intracellular introduction of a double stranded RNA (dsRNA) consisting of a sense RNA homologous to mRNA of a target gene and an antisense RNA having a sequence complementary to the sense RNA. Selective target gene silencing by RNAi has been increasingly focused and widely exploited as a simple gene knock-down or gene therapy, instead of the conventional inefficient gene disruption by homologous recombination. Chemically synthesized, short interfering RNA (hereinafter, referred to as "siRNA") and promoter-dependent expressed short hairpin RNA (hereinafter, referred to as "shRNA") show potent gene-silencing activity in vitro and in vivo. Compared to chemically synthesized siRNAs, however, promoter-dependent shRNAs give several advantageous features, including stable expression in cells and whole organisms, constitutive or inducible expression, and cell type-specific expression. shRNAs can be classified into two different kinds: the first-generation and second-generation shRNAs (FIG. 1). The first-generation shRNAs simply mimic chemically synthesized siRNAs, while the second-generation shRNAs are derived from natural microRNAs expressed in various organisms and show better functionality. The structural features of the second-generation shRNAs (FIG. 1) also allow more flexibility in selecting promoters and target shRNA sequences.

In case of siRNAs, mixed siRNAs acting on different sites are more frequently used to silence the single target gene than single siRNA acting on a single site, because mixed siRNAs acting on different sites show better silencing activity and various siRNAs can be easily prepared by chemical synthesis. Mixed siRNAs acting on different genes are also used to silence the multiple different genes, thereby appropriately silencing the various genes. In contrast, promoter-dependent shRNAs have been basically used as a mono-cistron, leading to silencing only one single target gene. Consequently, to silence multiple target genes using promoter-dependent shRNAs, delivery of the multiple shRNA expression constructs corresponding to the number of target genes is necessary. Thus, if the multiple target genes can be silenced using only one single shRNA expression construct, that approach will remarkably minimize the concerns arising from a use of multiple expression constructs, including a difficulty in the expression of equimolar amounts of shRNAs, high frequency of recombination between the repeated sequences of identical promoter, and quality control of shRNA expression constructs in the development of therapeutic agents such as gene therapy. Furthermore, that approach will allow easy and rapid analysis of the functions of the multiple target genes.

For the purpose of silencing single or multiple target genes by using one shRNA expression construct, the present inventors paid attention to the second-generation shRNAs, especially the mir-30-derived shRNA, since this shRNA has been better characterized for the structure and nuclease cleavage sites. The present inventors hypothesized that the structural features of mir-30 microRNA, importantly, structural flexibility associated with the leader sequence of microRNA might allow the creation of a cassette to express multi-cistronic transcript harboring multiple shRNAs. The present inventors have made an effort to demonstrate this hypothesis, thereby completing the present invention.

### SUMMARY OF THE INVENTION

An object of the present invention is to provide a multi-cistronic shRNA expression cassette comprising a promoter and two or more polynucleotides encoding shRNAs (short hairpin RNA) specific to target genes, which are operably linked to the promoter.

Another object of the present invention is to provide an expression vector comprising the expression cassette, a cell transduced with the expression vector, and a composite of the vector and a carrier.

Still another object of the present invention is to provide a method for suppressing various target genes of interest using the expression cassette.

Still another object of the present invention is to provide a composition for suppressing target genes comprising the expression vector.

### BRIEF DESCRIPTION OF THE DRAWINGS

FIG. 1 shows a structural comparison of siRNA, first-generation shRNA and second-generation shRNA, in which chemically synthesized siRNA and two different shRNAs are compared, and each of the nucleotide strands marked in the box and the color-reversed box indicates the sense and anti-sense sequences of a target gene, and in the second-generation shRNA, circled is the first nucleotide for the sense strand of the corresponding transcript, and the nuclease cleavage sites are marked by the lines of Drosha and Dicer;

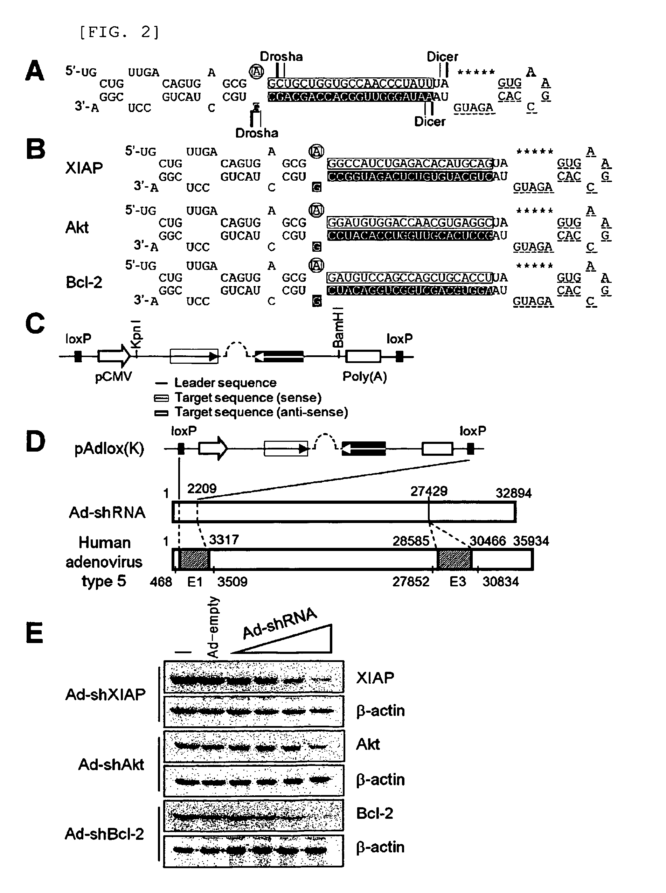

FIG. 2 shows functional tests of the individual shRNA expression constructs, in which
   A. shows mir-30-derived template shRNA sequence, this sequence except the nucleotide strands marked in the box and the color-reversed box is commonly used as a template for preparing all the shRNA expression constructs,
   B. shows shRNA sequences designed to silence human XIAP, Akt and Bcl-2, in which each of the nucleotide strands marked in the box and the color-reversed box indicates the sense and anti-sense sequences of a target gene,
   C. shows cloning of the insert DNA sequences corresponding to cognate shRNAs to the shuttle vector for adenovirus production,
   D. shows preparation of adenoviruses, in which shRNA-expressing adenoviruses were generated by Cre-lox recombination, and
   E. shows silencing activity tests for adenoviruses expressing mono-cistronic shRNA, in which HCT116 cells were infected with Ad-empty control virus or each of Ad-shRNAs, lysed 72 hours later and subjected to Western blot analysis, and the protein levels of β-actin were used as a loading control;

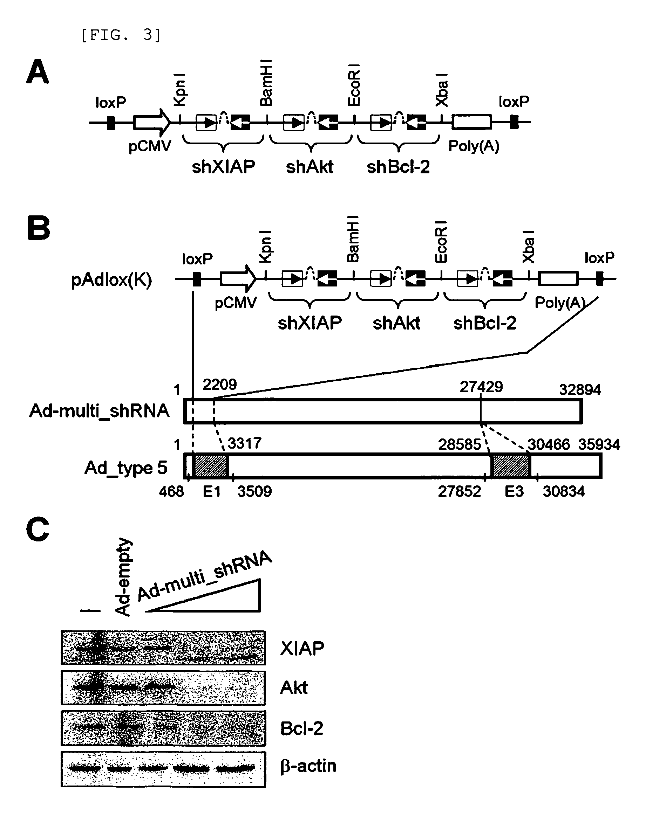

FIG. 3 shows gene silencing by the multi-cistronic shRNA expression vector, in which
   A. is a schematic drawing for pAdlox(K) shuttle vector harboring multiple shRNAs,
   B. shows preparation of adenovirus expressing multi-cistronic shRNAs, in which Ad-multi_shRNA was generated by Cre-lox recombination, and
   C. shows silencing activity tests for adenovirus expressing multi-cistronic shRNAs, in which HCT116 cells were infected with Ad-empty control virus or Ad-multi_shRNA, lysed 72 hours later and subjected to Western blot analysis, and the protein levels of β-actin were used as a loading control;

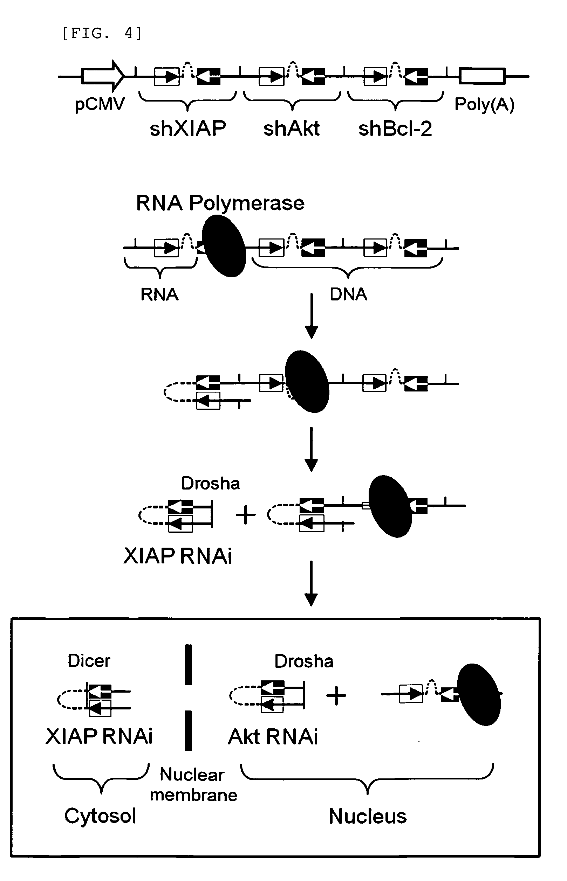

FIG. 4 shows a model for the process to produce different shRNAs from one multi-cistronic transcript, in which transcription and shRNA processing occur combined, each shRNA unit in one single transcript is processed in a stepwise manner, and once the first shRNA is transcribed, it forms a unique structure and is cleaved by Drosha that is a nuclease primarily localized in the nucleus, the Drosha-cleaved shRNA is exported to the cytosol and further processed by a cytosolic nuclease Dicer, and this one complete round continuously repeats itself to produce all the shRNAs encoded in the multi-cistronic transcript;

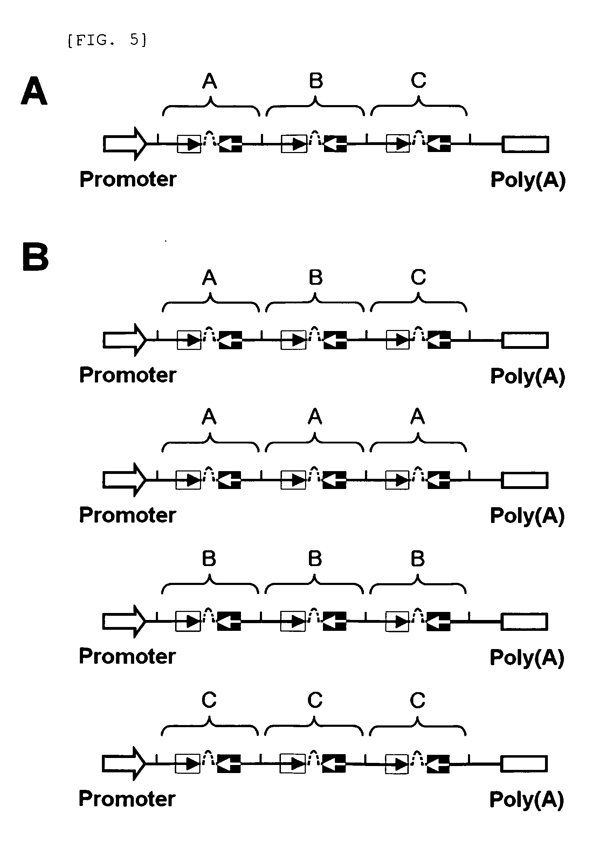

FIG. 5 shows constitution of multi-cistronic shRNA expression vector for silencing one single target gene, in which
   A. shows that each of the shRNAs in the multi-cistronic shRNA expression vector targets different sites of the single target gene, and
   B. shows that each of the shRNAs in the multi-cistronic shRNA expression vector targets different sites of the single target gene (ABC type), or same sites of the single target gene (AAA, BBB, CCC types);

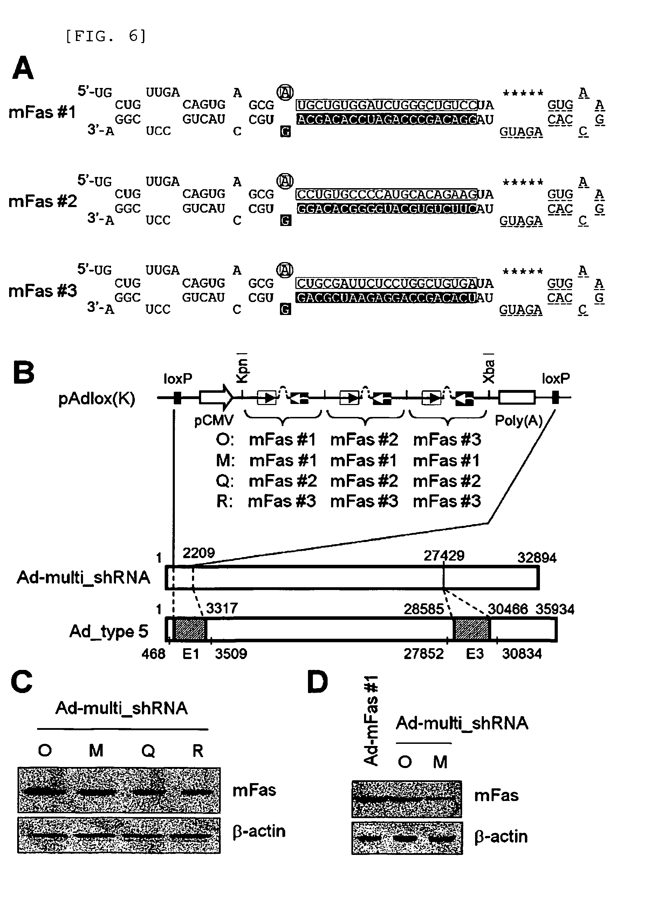

FIG. 6 shows constitution of multi-cistronic shRNA for a single target gene, that is, gene silencing by the multi-cistronic mFas shRNA expression vector, in which
   A. shows each shRNA sequence designed to silence mFas, each of the nucleotide strands marked in the box and the color-reversed box indicates the sense and anti-sense sequences of a target gene,
   B. shows preparation of adenovirus expressing multi-cistronic mFas shRNAs, in which Ad-multi_shRNAs (O, M, Q, R type) were generated by Cre-lox recombination,
   C. shows silencing activity tests for adenovirus expressing multi-cistronic shRNAs, in which Hepal-6 cells were infected with Ad-empty control virus or Ad-multi_shRNA (O: mFas#1-#2-#3) or Ad-multi_shRNA (M: mFas#1-#1-#1) or Ad-multi_shRNA (Q: mFas#2-#2-#2) or Ad-multi_shRNA (R: mFas#3-#3-#3), lysed 72 hours later and subjected to Western blot analysis, and the protein levels of β-actin were used as a loading control,
   D. shows comparison of silencing activity between adenovirus expressing mono-cistronic shRNAs and multi-cistronic shRNAs, in which Hepal-6 cells were infected with Ad-mFas #1 control virus or Ad-multi_shRNA (O: mFas#1-#2-#3) or Ad-multi_shRNA (M: mFas#1-#1-#1), lysed 72 hours later and subjected to Western blot analysis, and the protein levels of β-actin were used as a loading control; and

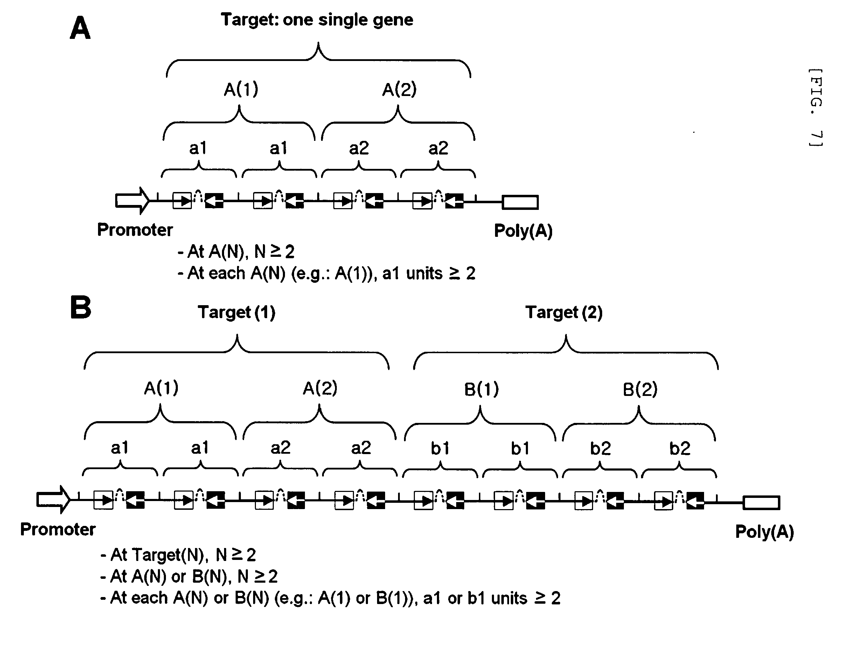

FIG. 7 shows constitution of multi-cistronic shRNA expression vector for silencing single or multiple target genes, in which A. shows constitution of multi-cistronic shRNA expression vector for silencing a single target gene, and shRNAs act to target two or more sites of the target gene (marked as A(N)), and shRNAs corresponding to each acting site are identical and the number is two or more (marked by a1, a2, etc.), and
   B. shows constitution of multi-cistronic shRNA expression vector for simultaneous silencing multiple target genes, and shRNAs act to target two or more sites (marked as A(N), B(N), etc.) of each target gene (marked as Target(1), Target(2), etc.), and shRNAs corresponding to each acting site are identical and the number is two or more (marked by a1, a2, etc.).

### DETAILED DESCRIPTION OF THE PREFERRED EMBODIMENTS

In one embodiment to achieve the above objects, the present invention relates to a multi-cistronic shRNA expression cassette comprising a promoter and two or more polynucleotides encoding shRNAs (short hairpin RNA) specific to target genes, which are operably linked to the promoter.

As used herein the term "promoter" refers to an untranslated DNA sequence usually located upstream of the coding region, which contains the binding site for RNA polymerase and initiates transcription of the gene downstream of the promoter into mRNA. In the expression cassette of the present invention, any promoter may be used, as long as it is able to initiate shRNA expression. Specifically, the promoter of the present invention may be a constitutive promoter which constitutively induces the expression of a target gene, or an inducible promoter which induces the expression of a target gene at a specific site and a specific time, and examples thereof include a U6 promoter, an H1 promoter, a CMV (cytomegalovirus) promoter, a SV40 promoter, a CAG promoter (Hitoshi Niwa et al., Gene, 108:193-199, 1991), a CaMV 35S promoter (Odell et al., Nature 313:810-812, 1985), a Rsyn7 promoter (US Patent Application NO. 08/991,601), a rice actin promoter (McElroy et al., Plant Cell 2:163-171, 1990), ubiquitin promoter (Christensen et al., Plant Mol. Biol. 12:619-632, 1989), and an ALS promoter(US Patent Application NO. 08/409,297). Additionally, any known promoter apparent to those skilled in the art, such as promoters disclosed in US Patent NOs. 5,608,149, 5,608,144, 5,604,121, 5,569,597, 5,466,785, 5,399,680, 5,268,463 and 5,608,142, may be used without limitation. Preferably, the promoter of the present invention may be a U6 promoter, an H1 promoter, or a CMV promoter. According to one preferred embodiment of the present invention, a CMV promoter may be used.

As used herein, the term "target gene" means a gene that is intended to be silenced, and it may be silenced by expression of shRNA specific to the target gene. The target gene in the present invention includes all genes, of which functions can be silenced by shRNAs specific to the target genes, and preferably XIAP (X-chromosome-linked inhibitor of apoptosis protein), Akt, Bcl-2 or Fas.

As used herein, the term "shRNA" means that a single strand RNA of 50 to 100 nucleotides forms a stem-loop structure in a cell, which contains a loop region of 5 to 30 nucleotides, and long complementary RNAs of 15 to 50 nucleotides at both sides of the loop region, which form a double-stranded stem by base pairing between the complementary RNAs; and additional 1 to 500 nucleotides included before and after each complementary strand forming the stem. shRNA is usually transcribed by RNA polymerase in a cell, and subsequently cleaved in the nucleus by Drosha, and the cleaved shRNA is exported from the nucleus to cytosol, and further cleaved in the cytosol by Dicer. Like siRNA, shRNA binds to the target mRNA in a sequence specific manner, thereby cleaving and destroying the target mRNA, and thus suppressing expression of the target mRNA. The shRNA-encoding polynucleotide of the present invention may be prepared from the microRNA-derived sequence, and the type of the microRNA is not particularly limited, and preferably one of microRNAs, mir-30-derived sequence. Specifically, the shRNA-encoding polynucleotide of the present invention may be prepared by linking a mir-30-derived leader sequence to a sense strand of target gene to be silenced. Preferably, shRNA for silencing XIAP of the present invention may have a sense strand of SEQ ID NO. 2, shRNA for silencing Akt may have a sense strand of SEQ ID NO. 3, shRNA for silencing Bcl-2 may have a sense strand of SEQ ID NO. 4 and shRNA for silencing mFas may have a sense strand of SEQ ID NO. 5, 6, or 7. The multiple and simultaneous silencing of XIAP, Akt, and Bcl-2 genes by the multi-cistronic shRNA of the present invention was observed in FIG. 3C of Example 3. In addition, the multiple and simultaneous silencing of mFas by the multi-cistronic shRNA of the present invention was examined in FIGs. 6C and 6D of Example 3.

In the above results, each shRNA in the multi-cistronic shRNAs expression construct used for knockdown acts on each mRNA of the corresponding target genes.

In the present invention, the shRNAs targeting mRNA of the target gene may act on a single site or different sites of a single target gene, and may act on a single site or different sites of each target gene in two or more different target genes. In order to efficiently silence the target gene using the multi-cistronic shRNA expression cassette of the present invention, it is preferable that the shRNAs act on two or more different sites of a single target gene, and the shRNAs corresponding to each site have the identical sequence and the number is two or more. To efficiently silence two or more different (multi) target genes, the method applied to the single target gene can be also applied to each target gene of the multiple target genes in the same manner.

As used herein, the term "operably linked" means that one nucleic acid fragment is linked to other nucleic acid fragment so that the function of each fragment in any possible binding combinations of the nucleic acid fragments is not affected by the other nucleic acid fragment. Moreover, the expression cassette of the present invention may further include any transcriptional initiation regulatory sequence and transcriptional termination regulatory sequence for regulation of the transcription. The operable linkage may be prepared using a genetic recombinant technique well known in the art, and site-specific DNA cleavage and ligation may be achieved using enzymes generally known in the art.

As used herein, the term "multi-cistronic" means that multiple cistrons, namely, multiple unit genes or multiple unit shRNA expression constructs, are linked to one promoter.

As used herein, the term "expression cassette" means a unit cassette capable of expressing shRNA, which includes a promoter and a microRNA leader sequence, in which the microRNA leader sequence includes a sense strand and an anti-sense strand of the target gene, and a sequence capable of forming a loop between the sense and anti-sense strands. Further, in the present invention, the expression cassette may be interchangeable with an expression construct. Preferably, a backbone of the cassette capable of expressing shRNA may be a microRNA mir-30-derived shRNA of SEQ ID NO. 1 as a template.

In order to construct a single multi-cistronic shRNA expression cassette of the present invention, the present inventors first examined the functionality of individual shRNAs in the specific Example. The present inventors selected the mir-30-derived sequence defined as SEQ ID NO. 1 (FIG. 2A) and prepared the corresponding DNA sequence by PCR to express each of the shRNAs for silencing human XIAP (X-chromosome-linked inhibitor of apoptosis protein), Akt, or Bcl-2 gene as a target (FIG. 2B). The present inventors cloned each of these DNA sequences into a CMV promoter-driven shuttle vector (FIG. 2C) and subsequently prepared corresponding adenoviruses, named Ad-shXIAP, Ad-shAkt and Ad-shBcl-2, respectively (FIG. 2D). The present inventors infected HCT116 cells with the prepared adenoviruses, and evaluated silencing activity of these adenoviruses by examining the protein levels of XIAP, Akt and Bcl-2 (FIG. 2E). As a result, Ad-shXIAP, Ad-shAkt and Ad-shBcl-2, but not control Ad-empty, silenced expression of the corresponding protein, confirming that each of the shRNA sequences appropriately acted to silence the target gene. In different cells (U-373MG) used to evaluate silencing activity of Ad-shXIAP, Ad-shAkt and Ad-shBcl-2, similar results were also obtained (data not shown).

The present inventors prepared a multi-cistronic shRNA expression construct using the above DNA sequences that functioned well (FIG. 3A). The present inventors prepared adenovirus, named Ad-multi_shRNA (FIG. 3B) and evaluated its silencing activity for the corresponding target genes. Ad-multi_shRNA effectively and simultaneously silenced expression of the multiple target genes (FIG. 3C), whereas the control Ad-empty did not. These results clearly show that delivery of the single expression cassette harboring multi-cistronic shRNAs is sufficient to silence multiple target genes.

Based on the above results, the present invention provides a multi-cistronic shRNA expression cassette as a powerful new tool for silencing multiple target genes by expressing one single multi-cistronic transcript. Based on the structural features, the cleavage sites in the mir-30-derived shRNA defined as SEQ ID NO. 1, and subcellular localizations of the nucleases Drosha and Dicer which are known to actually cleave the shRNA at the cleavage sites, the present inventors proposed a model for the process to generate different shRNAs from a single multi-cistronic transcript (FIG. 4). The model of the present invention suggests that the first shRNA (shXIAP) region is transcribed by RNA polymerase to generate shRNA corresponding thereto, and this shRNA is cleaved by Drosha localized in the nucleus, while the second shRNA is being transcribed. The Drosha-cleaved shRNA is exported into the cytosol and additionally cleaved by Dicer localized in the cytosol. This cycle continues onto reach the last shRNA (shBcl-2). This consecutive cleavage process by Drosha and Dicer nucleases likely prevents entanglement of a single multi-cistronic transcript and to generate shRNA in order. According to the model of the present inventors, the shRNA expression cassette can theoretically harbor numerous units of shRNAs, preferably two or more units of shRNAs, and more preferably four or more units of shRNAs. The maximum number of expressed shRNAs is the number corresponding to the maximum capacity and size that can be stably expressed by the expression vector.

Generally, a large number of genes exist in polymorphic forms according to differences between individuals. In particular, cancer cells are defective in tumor suppressor genes and DNA repair systems, and thus frequent mutations are observed. When mutations occur in the base sequence of a target gene, shRNAs used for knockdown lose their functions. In order to overcome the loss of function, it is required to target multiple regions rather than a single region as a target of shRNAs for one single target gene (FIG. 5A). In this regard, even though a certain shRNA in the multi-cistronic shRNA expression construct cannot perform its function due to mutations in the target gene, other shRNAs expressed from the same expression construct act on other regions, thereby effectively silencing the target gene. In addition, when the multi-cistronic shRNA is used to silence one single target gene, combination of shRNAs is very important. For example, when a multi-cistronic shRNA expression construct is composed of three shRNAs (A, B, C, hereinafter, referred to as "ABC") to silence one single target gene (FIG. 5B), mRNA of the target gene degraded and suppressed by A can also be simultaneously degraded and suppressed by B and C, and thus an AAA or BBB or CCC type can be practically better than the ABC type in terms of suppression efficiency. In order to demonstrate this, the present inventors selected the mir-30-derived sequence defined as SEQ ID NO. 1 (FIG. 2A) and prepared the corresponding DNA sequence by PCR to encode the shRNAs for silencing murine Fas (mFas) including SEQ ID NO. 5, SEQ ID NO. 6, SEQ ID NO. 7 (FIG. 6A). The present inventors cloned each of these DNA sequences into a CMV promoter-driven shuttle vector, and subsequently prepared corresponding adenoviruses, named Ad-mFas #1, Ad-mFas #2 and Ad-mFas #3, respectively. The present inventors infected Hepal-6 cells with the prepared adenoviruses, and evaluated silencing activity of these adenoviruses by examining the protein levels of mFas. As a result, Ad-mFas #1, Ad-mFas #2 and Ad-mFas #3, but not control Ad-empty, silenced expression of the corresponding protein, confirming that each of the shRNA sequences appropriately acted to silence the target gene (data not shown). Thereafter, the present inventors cloned the above mFas DNA sequences that functioned well into a CMV promoter-driven shuttle vector (FIG. 6B), and subsequently prepared corresponding adenoviruses, named Ad-multi_shRNA O, M, Q, and R (FIG. 6B). The present inventors infected Hepal-6 cells with the prepared adenoviruses, and evaluated silencing activity of these adenoviruses by examining the protein levels of mFas (FIG. 6C). As a result, M, Q, and R more effectively suppressed the expression of corresponding proteins than O. These results clearly show that the AAA or BBB or CCC type (FIG. 5) exhibits more efficient silencing activity than the ABC type in the case of silencing one single target gene using the multi-cistronic shRNA. Moreover, none of Ad-mFas #1, Ad-mFas #2, and Ad-mFas #3 showed more efficient silencing activity for mFas than the corresponding adenoviruses, named Ad-multi_shRNA O, M, Q, and R (FIG. 6D). These results indicate that the multi-cistronic shRNA composed of different shRNAs acting on the different sites of one single target gene will show more efficient silencing activity than single shRNA.

Taken together, the present inventors found out the optimal conditions for silencing a target gene using the multi-cistronic shRNA. That is, to silence one single target gene (FIG. 7A), the shRNAs may act on different sites (A(1), A(2).....A(N)), and the number may be two or more, and shRNAs corresponding to each site may be a single type (a1, a2.....aN), and the number may be two or more. That is, shRNAs corresponding to each acting site may be a single type and have the identical sequence. To silence multiple different target genes (FIG. 7B), the method applied to one single target gene can also be applied to each target gene of the multiple different target genes in the same manner.

Promoter-driven shRNAs can be stably expressed locally or globally in organisms. Stable expression of multi-cistronic shRNAs for multiple silencing of different regions of a single target gene (FIG. 7A) is expected to silence the single target gene more powerfully than that of individual mono-cistronic shRNAs. Thus, it is interesting to evaluate whether stable expression in whole organism of multi-cistronic shRNAs designed to silence one single target gene are functionally comparable to the gene knock-out. Multi-cistronic shRNAs stably expressed to silence multiple target genes will also become a powerful tool for analyzing the outcome resulting from the silenced multiple genes. Cell type-specific promoters used to express multi-cistronic shRNAs will facilitate analysis of cell type-specific silencing effects of the single or multiple target genes. From a therapeutic point of view, multi-cistronic shRNAs will be used as a tailored therapy which requires multiple target genes to be silenced. Therefore, the multi-cistronic shRNA of the present invention provides a versatile tool for various applications.

In another embodiment, the present invention relates to an expression vector, comprising the multi-cistronic shRNA expression cassette including a promoter and two or more polynucleotides encoding shRNAs (short hairpin RNA) specific to target genes, which are operably linked to the promoter. The multi-cistronic shRNA expression cassette is the same as described above.

The multi-cistronic shRNA expression construct can be introduced into a cell. To achieve this, the multi-cistronic shRNA expression construct may be included in a carrier that allows efficient introduction of the multi-cistronic shRNA expression construct into a cell. The carrier is preferably a vector, and the vector may be a viral vector or a non-viral vector. The viral vector may be exemplified by lentivirus, retrovirus, adenovirus, adeno-associated virus, herpes virus, and avipox virus, and preferably adenovirus, but is not limited thereto. The non-viral vector may be exemplified by plasmids. According to one preferred embodiment of the present invention, a shuttle vector, pAdlox (K) was used.

The multi-cistronic shRNA expression construct may be also introduced into a cell in various composite forms, together with PEG (polyethylene glycol), PEI (polyethyleneimine), chitosan, PEG-chitosan, DEAE-dextran, nucleoprotein, lipid, or peptide. To achieve this, the multi-cistronic shRNA expression construct may be included in the carrier that is a major component of the composites and allows efficient introduction of the multi-cistronic shRNA expression construct into a cell.

Further, it is preferable that the vector further includes a selection marker. As used herein, the term "selection marker" is required for easily selecting the cells transformed by introduction of the multi-cistronic shRNA expression construct. No particular limitations are imparted to the marker provided that it enables the introduction of the vector to be readily detected or measured. Typically, markers for conferring selectable phenotypes such as drug resistance, autotrophy, resistance to cytotoxic agents, or surface protein expression may be used. Examples of the markers include GFP (green fluorescent protein), puromycin, neomycin (Neo), hygromycin (Hyg), histidinol dehydrogenase gene (hisD), and guanine phosphosribosyltransferase (Gpt), and preferably GFP (green fluorescent protein), neomycin or puromycin marker.

In still another embodiment, the present invention relates to a cell transduced with the expression vector.

As used herein, the term "introduction" is intended to mean the delivery of foreign DNA into cells through transfection or transduction. Transfection may be carried out using various methods well known in the art, including calcium phosphate-DNA co-precipitation, DEAE-dextran-mediated transfection, polybrene-mediated transfection, electroporation, microinjection, liposome fusion, lipofectamine transfection, and protoplast fusion. Transduction refers to a process whereby an object is transferred to cells using viral particles by means of infection. Preferably, transduction may be achieved via a composite with a carrier of PEG, chitosan, PEG-chitosan, DEAE-dextran, nucleoprotein, lipid or peptide.

From a therapeutic point of view, the cells transduced by introduction of the expression vector can be used in a patient-tailored therapy by introduction of the multi-cistronic shRNA expression construct capable of selectively silencing the target gene.

In addition, if cell type-specific promoters are chosen, they will facilitate easy analysis of cell type-specific silencing effects of the single or multiple target genes, and thus the transduced cells can be used as a model for analyzing the cell type-specific silencing effects.

In still another embodiment, the present invention relates to a composite with a carrier selected from the group consisting of PEG, PEI, chitosan, PEG-chitosan, DEAE-dextran, nucleoprotein, lipid, and peptide for the introduction of the vector into cells.

In still another embodiment, the present invention relates to a method for suppressing various target genes, comprising the steps of (a) preparing an expression vector including the multi-cistronic shRNA expression cassette; and (b) introducing the prepared vector into a cell.

In still another embodiment, the present invention relates to a composition for suppressing target genes, comprising the multi-cistronic shRNA expression cassette including the above described promoter and two or more polynucleotides, which are operably linked to the promoter.

The composition for suppressing target genes may further include a pharmaceutically acceptable carrier, and formulated together with the carrier. As used herein, the term "pharmaceutically acceptable carrier" refers to a carrier or diluent that does not cause significant irritation to an organism and does not abrogate the biological activity and properties of the administered compound. For use in liquid formulations of the composition of the present invention, the pharmaceutically acceptable carrier is preferably suitable for sterilization and the living body. It may be formulated with one or more selected from saline, sterile water, Ringer's solution, buffered saline, albumin injection, dextrose solution, maltodextrose solution, glycerol, ethanol and combinations thereof, and if necessary, in combination with other conventional additives including antioxidants, buffer, bacteriostatic agents, etc. Alternatively, the composition of the present invention may be formulated into injectable preparations such as aqueous solution, suspension and emulsion, pills, capsules, granules, or tablets, together with diluents, dispersants, surfactants, binders and lubricants.

The composition for target gene suppression comprising the expression vector and the pharmaceutically acceptable carrier may be formulated into any dosage form including the composition as an active ingredient, whether oral or parenteral. The pharmaceutical formulations of the present invention may be administered via oral, rectal, nasal, topical (including bolus and sublingual), transdermal, vaginal, or parenteral (including intramuscular, subcutaneous and intravenous) routes or by inhalation or insufflation.

Examples of the oral dosage forms including the composition of the present invention as an active ingredient include tablets, troches, lozenges, water-soluble or oil suspensions, powders, granules, emulsions, hard or soft capsules, syrups or elixirs. For tablet or capsule formulations, useful are additives including a binder, such as lactose, saccharose, sorbitol, mannitol, starch, amylopectin, cellulose or gelatin, an excipient such as dicalcium phosphate, a disintegrant such as corn starch or sweet potato starch, and a lubricant, such as magnesium stearate, calcium stearate, sodium stearyl fumarate, or polyethylene glycol wax. In addition to these additives, a liquid carrier such as fat oil may be used for capsule formulations.

For use in parenteral administration, the composition of the present invention may be formulated into injectable preparations via subcutaneous, intravenous or intramuscular routes, suppositories, or sprays via inhalation, such as aerosols. Injectable preparations may be prepared by mixing the composition of the present invention with a stabilizer or buffer in water to give solutions or suspensions which are packaged in unit dosages such as ampules or vials. For suppositories, the composition of the present invention may be formulated into a composition for rectal administration such as suppository including a conventional base such as cocoa butter or glyceride, or enema. Upon formulation into spray such as aerosol, the composition of the present invention may be blended with a propellant to be formulated in a form of a water-dispersed concentrate or a wet powder.

The composition for suppressing target genes, namely, the multi-cistronic shRNA expression vector may be a cell expressing the expression vector. The cell may be a cell capable of transiently or stably expressing the multi-cistronic shRNA expression vector for target gene suppression.

Hereinafter, the present invention will be described in more detail with reference to Examples. However, these Examples are for illustrative purposes only, and the invention is not intended to be limited by these Examples.

### Example 1: Preparation of shRNA expression constructs and corresponding adenovirus

The DNA sequences corresponding to each of the shRNAs for human XIAP, Akt and Bcl-2 and murine Fas (mFas) were prepared by overlapping PCR. The template DNAs containing the sense or anti-sense shRNA sequence were chemically synthesized and amplified by PCR using a common primer set harboring the different restriction enzyme sites. The PCR products were digested with restriction enzymes and cloned into the corresponding sites of the pAdlox(K), which is a shuttle vector for adenovirus production. The insert sequences were verified by nucleotide sequencing analysis.

E1/E3-doubly deleted replication-incompetent adenoviruses were prepared through Cre-lox recombination according to a method described in Jeong, M. et al. PLoS ONE 4, E4545 (2009). Adenoviruses were purified using an adenovirus purification kit, Ad EZ-Prep (Genenmed Inc., Seoul, Korea).

As a result, each DNA sequence of the shRNAs for silencing human XIAP, Akt, Bcl-2 (FIG. 2B) or mFas gene was prepared (FIG. 6A), based on the mir-30-derived base sequence of SEQ ID NO. 1 (FIG. 2A). The present inventors cloned each of these DNA sequences into a CMV promoter-driven shuttle vector (XIAP, Akt, Bcl-2: FIG. 2C, mFas: data not shown), and subsequently prepared corresponding adenoviruses, named Ad-shXIAP, Ad-shAkt and Ad-shBcl-2, respectively (FIG. 2D).

Further, the present inventors prepared a multi-cistronic shRNA expression construct using each DNA sequence that functioned well (FIG. 3A, FIG. 6A). By using this, they prepared an adenovirus, named Ad-multi_shRNA (FIG. 3B, FIG. 6B).

### Example 2: Cell culture

HCT116, U373-MG, or Hepa1-6 cells were originally obtained from American Type Culture Collection (ATCC) (Rockville, MD). The cells were cultured in McCoy's 5A (HCT116) and DMEM (U373-MG and Hepa1-6), respectively.

### Example 3: Western blot analysis and Target gene silencing

In order to examine silencing activity of each shRNA and multi-cistronic shRNA expression construct, the present inventors infected the HCT116, U373-MG or Hepa1-6 cells with the adenoviruses prepared in Example 1, and then observed expression patterns of each target gene.

HCT116, U373-MG, or Hepa1-6 cells were incubated with adenoviruses for 4 hours. After changing the culture mediums to a fresh one, cells were further incubated for 72 hours. Cells were harvested and lysed in a RIPA buffer, followed by Western blot analysis. Antibodies for detecting XIAP (#2045, Cell signaling Technology), Akt (LF-PA0166, Ab Frontier), Bcl-2 (sc-492, Santa Cruz Biotechnology), mFas (610197, BD Pharmingen), and β-actin (LF-PA0066, Ab Frontier) were used.

As a result, expression of the proteins corresponding to Ad-shXIAP, Ad-shAkt, Ad-shBcl-2, Ad-mFas #1, Ad-mFas #2 and Ad-mFas #3 was silenced (FIG. 2E, Ad-mFas: data not shown). However, the control Ad-empty did not suppress their expression. These results indicate that each of the shRNA sequences appropriately acted to silence expression of the target genes.

In addition, Ad-multi_shRNA effectively and simultaneously silenced expression of the corresponding target genes (FIG. 3C, FIG. 6C) whereas the control Ad-empty did not. These results of the present inventors clearly show that delivery of single expression cassette harboring multi-cistronic shRNAs is sufficient to silence multiple target genes.

Moreover, with respect to the mFas-silencing Ad-multi_shRNA prepared to silence a single target gene, Ad-multi_shRNA (M: mFas#1-#1-#1), Ad-multi_shRNA (Q: mFas#2-#2-#2) and Ad-multi_shRNA (R: mFas#3-#3-#3) including three of the identical sequence to silence the identical region showed more efficient silencing activity for mFas than the Ad-multi_shRNA (O: mFas#1-#2-#3) prepared to silence the different regions within the target gene (FIG. 6C), and much more efficient silencing activity than Ad-mFas #1 (FIG. 6D).

These results indicate that when multiple shRNAs having the identical sequence to act on the identical region are included in one single multi-cistronic shRNA, it can silence the target genes more effectively.

### Effect of the invention

Multi-cistronic shRNAs expression construct stably expressed to silence multiple target genes will also become a powerful tool for analyzing the outcome resulting from the silenced multiple genes. Cell type-specific promoters used to express multi-cistronic shRNAs will facilitate analysis of cell type-specific silencing effects of the single or multiple target genes. From a therapeutic point of view, the multi-cistronic shRNA expression construct will be used as a tailored therapy which requires multiple target genes to be silenced. Therefore, the multi-cistronic shRNA expression construct of the present invention can provide a versatile tool for various applications.

## Claims

1. A multi-cistronic shRNA expression cassette comprising a promoter and two or more polynucleotides encoding shRNAs (short hairpin RNA) specific to target genes, which are operably linked to the promoter.

2. The multi-cistronic shRNA expression cassette according to claim 1, wherein the two or more polynucleotides are polynucleotides encoding shRNAs specific to a single site or different sites of one target gene.

3. The multi-cistronic shRNA expression cassette according to claim 1, wherein the two or more polynucleotides are polynucleotides encoding shRNAs specific to a single site or different sites of each target gene of multiple target genes.

4. The multi-cistronic shRNA expression cassette according to claim 1, wherein the two or more polynucleotides have the identical sequence.

5. The multi-cistronic shRNA expression cassette according to claim 1, wherein the promoter is selected from the group consisting of a U6 promoter, a H1 promoter, and a CMV promoter.

6. The multi-cistronic shRNA expression cassette according to claim 1, wherein the target gene is selected from the group consisting of XIAP, Akt, Bcl-2, and Fas.

7. The multi-cistronic shRNA expression cassette according to claim 1, wherein the shRNA is derived from the microRNA mir-30 of SEQ ID NO. 1.

8. An expression vector comprising the multi-cistronic shRNA expression cassette as in one of claims 1 - 7.

9. The expression vector according to claim 8, wherein the expression vector is a plasmid.

10. The expression vector according to claim 8, wherein the expression vector is selected from the group consisting of lentivirus, retrovirus, adenovirus, adeno-associated virus, herpes virus, and avipox virus.

11. A cell transduced with the expression vector of claim 8.

12. A composite of the vector of claim 8 and a carrier selected from the group consisting of PEG (polyethylene glycol), PEI (polyethyleneimine), chitosan, PEG-chitosan, DEAE-dextran, nucleoprotein, lipid, and peptide for the transduction of the vector into a cell.

13. A method for suppressing various target genes in a cell, comprising:
(a) preparing the expression vector of claim 8; and
(b) introducing the prepared vector into the cell.

14. A composition for suppressing target genes, comprising the expression vector of claim 8.
